# EUROPÄISCHE PATENTSCHRIFT

(11) **EP 3 800 275 B1**
(45) Veröffentlichungstag und Bekanntmachung des Hinweises auf die Patenterteilung: **31.07.2024**
(21) Anmeldenummer: 20195757.8
(22) Anmeldetag: 11.09.2020
(51) Int. Cl.: C14B 17/00, D06H 3/08, G01N 21/00, G01N 21/88, G01N 33/44, G06T 7/00

(54) **VORRICHTUNG UND VERFAHREN ZUR QUALITÄTSEINSTUFUNG VON TIERHÄUTEN**
DEVICE AND METHOD FOR ASSESSING THE QUALITY OF ANIMAL SKINS
DISPOSITIF ET PROCÉDÉ DE CLASSIFICATION QUALITATIVE DES PEAUX D'ANIMAUX

(30) Priorität: 01.10.2019 DE 102019006835
(43) Veröffentlichungstag der Anmeldung: 07.04.2021
(73) Patentinhaber: GRAMMER AG, 92289 Ursensollen (DE)
(72) Erfinder: Mauthner, Hermann, 93161 Sinzing (DE); Bauer, Ludwig, 92269 Fensterbach (DE)
(74) Vertreter: Roche, von Westernhagen & Ehresmann

(56) Entgegenhaltungen:
- EP-A1- 2 787 485
- EP-A1- 2 835 430
- WO-A1-2011/085935
- DE-A1- 102010 021 274
- JP-U- 3 220 455
- US-A- 6 157 730

## Beschreibung

Die Erfindung betrifft ein Verfahren zur Qualitätseinstufung von Tierhäuten.

Ein solches Verfahren ist aus offenkundiger Vorbenutzung bekannt. Bei dem bekannten Verfahren treffen Tierhäute von unterschiedlicher Größe und Qualität beim Kunden im Wareneingang ein. Diese werden stichprobenartig auf Fehler geprüft und in ein Lager verbracht. Bei Bedarf werden die Tierhäute dem Lager entnommen und es findet eine Qualitätskontrolle jeder einzelnen Tierhaut statt. Die Fehlstellen der Tierhaut werden markiert und die Tierhaut mit den Fehlstellen wird datenmäßig erfasst. Daraufhin findet ein sogenanntes "Nesting" statt, d.h. die benötigten Flächenzuschnitte werden auf der Tierhaut derart angeordnet, dass eine größtmögliche Ausnutzung erreicht wird. Danach findet der Zuschnitt statt, der Abfall wird von den Zuschnitten getrennt und die Zuschnitte werden zur Weiterverarbeitung in ein Zwischenlager überführt.

Da die Teile erst bei Bedarf aus dem Lager entnommen wurden, fand rasch nach der Qualitätskontrolle der jeweiligen Tierhaut die Weiterverarbeitung statt. Dem Lieferanten konnte daher nicht mehr nachgewiesen werden, dass die betreffende Tierhaut nicht der verkauften Qualität entsprach. Es musste daher eine hohe Anzahl von Tierhäuten vorgehalten werden, um Gutteile in ausreichender Menge produzieren zu können, was hohe Lagerkosten hervorrief.

Die US 6,157,730 betrifft eine Vorrichtung zum visuellen Überprüfen, insbesondere von Lederhäuten. Die Vorrichtung umfasst Mittel zum Spannen der Haut sowie eine Kamera zum Detektieren der Fehler. Des Weiteren ist eine Lichtquelle vorgesehen, welche die Haut beleuchtet. Auf diese Weise können Fehler dargestellt und erkannt werden, indem sie eine andere Helligkeit in Bezug auf das Material aufweisen, welches fehlerfrei ist.

Das Leder wird - je nach Schwere des Fehlers - in Qualitätsstufen eingeteilt.

Nachdem eine Karte der Lederhaut mit den Fehlern erzeugt wurde, wird die Lederfläche mit einer Schablone gerastert, wobei die von der Schablone erfassten Bereiche, die einen Fehler aufweisen, nicht verwendet werden. Die Methode liefert eine Information über die verwertbare Fläche der Lederhaut. Die Information kann an den Produzenten weitergegeben werden.

Die EP 2 787 485 A1 betrifft ein Verfahren zur automatischen Fehlerstellenerkennung bei biegeschlaffen Körpern, insbesondere bei Tierhäuten. Dabei wird mittels einer CCD-Kamera eine Aufnahme mindestens eines Teils des Körper erstellt. Die Aufahme wird in mehrere Bilder unterteilt. Die Bilder werden dann anhand von Grauwertstufen oder Farbwerten analysiert. Dabei fließen Nachbarschaftsbeziehungen in die Beurteilung ein.

Da das Nesting firmeninternes Know-How darstellt, sollte der Lieferant nicht über das Nesting mit den tatsächlichen Zuschnitten, die im Folgenden als Real-Geometrien bezeichnet werden, informiert werden.

Es war daher Aufgabe der Erfindung ein Verfahren zu schaffen, mit welchem gegenüber dem Lieferanten der Tierhäute ein objektiver Qualitätsnachweis für jede Haut erbracht werden kann, wobei keine Informationen über das Nesting mit Real-Geometrien offenbart werden müssen.

Die Aufgabe wurde gelöst mit den Merkmalen des Anspruchs 1.

Nach dem erfindungsgemäßen Verfahren wird die jeweilige Tierhaut nach Größe und nach Fehlstellen erfasst. Das bedeutet, dass die Flächenform, die Flächengröße und die Flächenposition gespeichert werden. Es wird eine Topographie der Gutflächen und der Schlechtflächen der Tierhaut festgelegt. Schlechtflächen sind Flächen, die nicht im späteren Tierhautzuschnitt enthalten sein dürfen. Das können Flächen z.B. sein, die beim Gerbvorgang Schaden genommen haben oder die aufgrund von natürlichen Gegebenheiten vorkommen, wie nicht akzeptable Naturmerkmale oder Produktionsfehler oder auch Öffnungen im Leder. Erstellung einer Topografie bedeutet im Sinne der Erfindung, dass die Schlechtstellen in ihrer Flächenform, Flächengröße und Flächenposition relativ zu der Flächenform, Flächengröße und Flächenposition der Tierhaut erfasst werden. Auf diese Weise kann auch die Flächenform, Flächengröße und Flächenposition der Gutflächen berechnet werden. Die ermittelten Daten werden in einem Datenspeicher gespeichert.

Die Erfassung der Schlechtflächen kann z.B. durch Inaugenscheinnahme und anschließende manuelle Markierung mittels eines Stiftes erfolgen. Dann wird z.B. mittels einer Vorrichtung enthaltend einen Auflagetisch für die Tierhaut, eine Kamera und einen mit der Kamera über ein Datenkabel verbundenen Computer ein Bild erzeugt aus welchem eine Software die Gutflächen und die markierten Schlechtflächen sowie deren Anordnung ermittelt und speichert.

Alternativ kann die Ermittlung der Schlechtflächen auch mittels einer Software der vorerwähnten Vorrichtung erfolgen, nachdem von der Tierhaut mit einer Kamera ein Bild erzeugt wurde. In diesem Fall registriert die Software die Schlechtstellen relativ zu der Tierhaut automatisch, und ermittelt sowohl Gutflächen, als auch Schlechtflächen sowie deren Anordnung und speichert die Daten. Die Qualifikation der Fehlerstellen erfolgt hierbei automatisch durch eine Software.

Danach wird ein Standard-Nesting durchgeführt. D.h., eine Standard-Geometrie in Form einer Fläche, d.h. einer zweidimensionalen Geometrie, wird auf der fehlerfreien Gutfläche der Tierhaut derart sooft wie möglich ohne Überschneidung der Standard-Geometrien angeordnet, dass eine möglichst große Ausnutzung der Tierhaut erfolgt. Für ein Standard-Nesting wird also immer dieselbe Standard-Geometrie verwendet. Grundsätzlich ist es möglich, die verwendete Geometrie aus allen erdenklichen Geometrien frei zu wählen.

Vorteilhaft ist es, wenn die Standard-Geometrie eine Flächengröße aufweist, die beim Standard-Nesting vergleichbare Werte liefert, wie die Geometrien, die später bei dem Nesting der tatsächlichen Zuschnitte verwendet werden. D.h., dass die Standard-Geometrie eine repräsentative Bauteilgeometrie darstellt.

Die Größe der Standard-Geometrie ist grundsätzlich frei wählbar, sollte aber, wie oben dargelegt, einen repräsentative Größe haben, wie aus dem Begriff "repräsentative Bauteilgeometrie" hervorgeht. Die Standard-Geometrie stellt z.B. eine abstrahierte, von den Realgeometrien abweichende Form dar. D.h., dass die Standard-Geometrie von den Real-Geometrien der verwendeten Teilezuschnitte bezüglich seiner Form und / oder bezüglich seine Flächengröße abweicht. Als Real-Geometrie wird die Geometrie bezeichnet, die später in einem Real-Nesting dem tatsächlichen Zuschnitt entspricht. Nach dem Standard-Nesting mit der Standard-Geometrie findet also noch ein Real-Nesting mit Real-Geometrie statt. Das heißt eine, mehrere gleiche oder mehrere unterschiedliche Real-Geometrien in Form einer Fläche werden auf der fehlerfreien Gutfläche der Tierhaut derart ohne Überschneidung angeordnet, dass eine möglichst große Ausnutzung der Tierhaut erfolgt. Die Zeit zwischen dem Standard-Nesting und dem Real-Nesting ist nicht wesentlich.

Bezüglich der Flächengröße ist die Standard-Geometrie z.B. an die Fläche der größten Teile eines Cut-Kits angenähert. Als Cut-Kit wird dabei die Gesamtheit der Zuschnitte bezeichnet, welche für ein zu fertigendes Bauteil oder mehrere zu fertigende Bauteile, wie z.B. die Bezüge unterschiedlicher Kopfstützen, benötigt werden. Für Bauteile mit stark abweichender Flächengröße, muss ggf. eine andere Standard-Geometrie verwendet werden (z.B. für Bezüge von Fahrzeugsitzen).

D.h., der Lieferant und ggf. Dritte können von den genesteten Standard-Geometrien nicht auf die später genesteten Real-Geometrien rückschließen. Dennoch erlaubt das erfindungsgemäße Verfahren eine zuverlässige und objektive Auskunft über die Qualität der jeweiligen Tierhaut. Diese Qualitätseinstufung ist auch dann noch belegbar, wenn die Tierhaut bereits verarbeitet wurde und physisch nicht mehr zur Verfügung steht.

Die Standard-Geometrie kann z.B. von einem Viereck, insbesondere einem Rechteck, einem Dreieck oder einem Kreis, oder durch zusammengesetzte Polygone verschiedener Geometrie gebildet sein.

Ein Bild, enthaltend die Tierhaut einschließlich der enthaltenen Gutflächen und Schlechtflächen sowie ggf. des Standard-Nesting-Ergebnisses wird z.B. gemäß einer Ausführungsform gespeichert.

Es wird z.B. das Verhältnis von Tierhautfläche zu der Anzahl der auf der Tierhaut beim Standard-Nesting angeordneten Standard-Geometrien gebildet. Dieses Verhältnis oder mit anderen Worten dieser Quotient bildet einen Wert, welcher der Ermittlung der Qualitätsstufe dient.

Die Tierhaut wird unter Verwendung des Standard-Nesting-Ergebnisses in eine Qualitätsstufe eingeordnet. Die Einstufung kann erfolgen, indem das zuvor erwähnte Verhältnis von Tierhautfläche zu der Anzahl der auf der Tierhaut beim Standard-Nesting angeordneten Standard-Geometrien verglichen wird mit einem Diagramm, welches für die jeweiligen Tierhautgrößen und die Anzahl der auf der Tierhaut angeordneten Standard-Geometrien Qualitätsintervalle enthält. Die Qualitätsintervalle sind frei wählbar.

Die ermittelte Qualitätsstufe wird der Haut zugeordnet. Die Haut bekommt z.B. einen Code, unter welchem ein Datensatz hinterlegt ist, in welchem die Qualitätsstufe der Tierhaut und z.B. ein Bild der Tierhaut mit Darstellung der Schlechtflächen und dem Standard-Nesting-Ergebnis gespeichert ist. Weitere tierhautbezogenen Daten können enthalten sein.

Tierhäuten, welche die Mindest-Qualitätsanforderungen nicht erfüllen, werden z.B. ausgesondert und z.B. an den Lieferanten zurückgesendet.

Gemäß einer Ausführungsform werden die Tierhäute, die dem Wert der Mindest-Qualitätsanforderungen genügen, eingelagert, z.B. separat nach Qualitätskategorie. Die Menge z.B. gemessen in m² je Qualitätsstufe wird von einer Software batchweise erfasst und die Batches erhalten je nach Qualität der Lieferung den vorher festgelegten Preis für eine A-Qualität (100 %-Bereich) oder werden auf- oder abgepreist. Als Batch wird eine Einheit, enthaltend eine bestimmte Anzahl von Tierhäuten, bezeichnet. Hierbei kann die Ab- bzw. Aufpreisung batchweise z.B. über die m² je Qualitätsgrad und Batch mit 25 % Aufpreisung bei AA-Qualität, 25 %, Abpreisung bei B-Qualität oder 50 % Abpreisung bei C-Qualität erfolgen. Die jeweiligen Auf- bzw. Abpreisungen und Prozentsätze können im Bewertungssystem für die Quotienten von Tierhautfläche zu der Anzahl der auf der Tierhaut beim Standard-Nesting angeordneten Standard-Geometrien hinterlegt sein. Für das spätere stattfindende Real-Nesting mit den Real-Geometrien kann dann die Tierhaut mit den gewünschten Eigenschaften dem Lager entnommen werden.

Die Erfindung betrifft gemäß einem zweiten Aspekt eine Vorrichtung zur Durchführung des Verfahrens.

Aus offenkundiger Vorbenutzung ist es bekannt, Fehler in der Tierhaut manuell zu kennzeichnen und aus dem Ergebnis subjektiv eine Bewertung durchzuführen. Die gekennzeichneten Stellen konnten mittels einer Kamera und einer mit der Kamera verbundenen Computer-Software als Schlechtstellen erkannt werden, so dass ein Bild einer Tierhaut in Gutflächen und Schlechtflächen unterteilt und ein Nesting mit den gewünschten Zuschnitten durchführbar war.

Es war Aufgabe der Erfindung eine Vorrichtung zu schaffen, mittels welcher eine Qualitätseinstufung der Tierhäute möglich ist, ohne Dritten Informationen über das Nesting mit den tatsächlichen Zuschnitten zu geben.

Die Aufgabe wurde gelöst durch eine Vorrichtung mit den Merkmalen des Anspruchs 11.

Die Vorrichtung umfasst einen Tisch zum Auflegen einer Tierhaut, eine Kamera zur Erstellung eines Bildes der Tierhaut und einen Computer mit wenigstens einer darauf betriebenen Software. Der Computer ist mit der Kamera über ein Datenkabel verbunden. Mittels wenigstens einer auf dem Computer betriebenen Software sind die von der Kamera erzeugten Bilddaten derart verarbeitbar, dass Gutflächen und Schlechtflächen der Tierhaut in Bezug auf ein Gesamtbild der Tierhaut erfassbar sind und ein Nesting durchführbar ist.

Erfindungsgemäß ist mittels des Computers und der Software ein Standard-Nesting durchführbar, bei welchem eine Standard-Geometrie möglichst oft auf der Gutfläche der Tierhaut angeordnet wird, so dass die Gutfläche maximal ausgenutzt wird. Um Wiederholungen zu vermeiden wird, was die Definition des Begriffs "Standard-Geometrie" und die möglichen Ausführungsformen der Standard-Geometrie anbelangt, auf die Beschreibung des erfindungsgemäßen Verfahrens verwiesen.

Es sei aber auf folgendes hingewiesen: Die Standard-Geometrie stellt eine abstrahierte, von den Realgeometrien abweichende Form dar. D.h., dass die Standard-Geometrie von den Real-Geometrien der verwendeten Teilezuschnitte bezüglich seiner Form und / oder bezüglich seine Flächengröße abweicht.

Gemäß einer Ausführungsform sind mittels Vorrichtung die Schlechtflächen unmittelbar aus dem erstellten Bild erkennbar oder alternativ ist eine manuelle Kennzeichnung der Schlechtflächen aus dem erstellten Bild erkennbar und nach Form, Größe und Position relativ zu der Form, Größe und Position der Fläche der Tierhaut anordenbar und speicherbar.

Gemäß einer Ausführungsform ist mittels des Computers und der Software ein Quotient aus der Anzahl der im Standard-Nesting, also dem Nesting mit Standard-Geometrien, auf der Gutfläche der Tierhaut angeordneten Standard-Geometrien und der Größe der Tierhaut errechenbar. Dieser Quotient dient der späteren Qualitätseinstufung der Tierhaut.

Die Größe der Tierhaut kann z.B. ebenfalls von der Vorrichtung ermittelbar sein oder alternativ von dem Lieferanten angegeben werden.

Einer weiteren Ausführungsform gemäß ist mittels der Computer-Software der vorerwähnte Quotienten mit einem Werteschema vergleichbar, in welchem die Qualitätsbereiche in Abhängigkeit der Hautgröße hinterlegt sind und die Qualitätsstufe auf diese Weise ermittelbar.

Ein Ausführungsbeispiel der Erfindung ist in der nachfolgenden Figurenbeschreibung, auch unter Bezugnahme auf die schematischen Zeichnungen, beispielhaft beschrieben. Dabei werden der Übersichtlichkeit halber gleiche oder vergleichbare Teile oder Elemente oder Bereiche mit gleichen Bezugszeichen, teilweise unter Hinzufügung kleiner Buchstaben, bezeichnet.

Merkmale, die nur in Bezug auf ein Ausführungsbeispiel beschrieben, dargestellt oder offenbart sind, können im Rahmen der Erfindung auch bei jedem anderen Ausführungsbeispiel der Erfindung vorgesehen werden. Derartig geänderte Ausführungsbeispiele sind - auch wenn sie in den Zeichnungen nicht dargestellt sind - von der Erfindung mit umfasst.

Alle offenbarten Merkmale sind für sich erfindungswesentlich. In die Offenbarung der Anmeldung wird hiermit auch der Offenbarungsinhalt der beschriebenen Vorrichtungen des Standes der Technik inhaltlich vollumfänglich mit einbezogen, auch zu dem Zweck, einzelne oder mehrere Merkmale der dort offenbarten Gegenstände in einen oder in mehrere Ansprüche der vorliegenden Anmeldung mit aufzunehmen. Auch solche geänderten Ausführungsbeispiele sind - auch wenn sie in den Zeichnungen nicht dargestellt sind - von der Erfindung mit umfasst.

Die schematischen Fig. zeigen:
- Fig. 1: ein Ablaufdiagramm mit einem beispielhaften Ablauf des erfindungsgemäßen Verfahrens,
- Fig. 2: ein Flussdiagramm der Qualitätsbewertung mehrerer beispielhafter Tierhäute mit dem erfindungsgemäßen Verfahren,
- Fig. 3: eine Vorrichtung zur Aufnahme der Fläche der Tierhaut sowie zur Bestimmung der Schlechtflächen und der Gutflächen und zur Durchführung des Standard-Nestings,
- Fig. 4a: eine Darstellung eines Bildes der Tierhaut mitsamt den gekennzeichneten Schlechtflächen
- Fig. 4b: eine Darstellung eines Bildes der Tierhaut gemäß Fig. 4a, wobei das Bild zusätzlich das Standard-Nesting-Ergebnis enthält,
- Fig. 5: ein Bewertungs-Feld, welchem der Qualitätsbereich der Haut anhand von Brutto-Hautfläche und Anzahl der genesteten Standard-Geometrien entnommen werden kann,
- Fig. 6: Darstellung eines Batches von 7 Tierhäuten mit unterschiedlichen Brutto-Hautflächen und unterschiedlichen Qualitätsstufen.

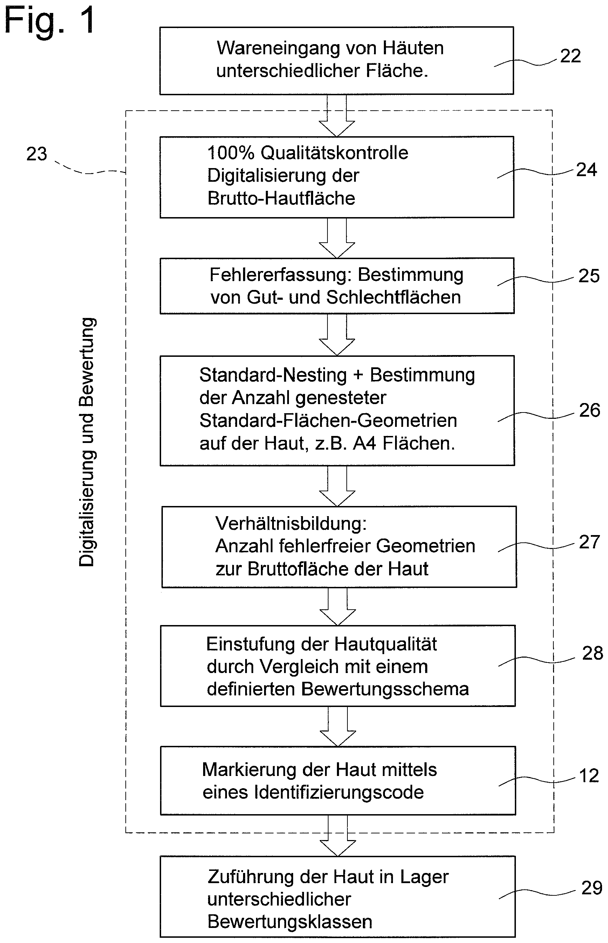

In Fig. 1 ist ein möglicher Ablauf des Verfahrens zur Ermittlung der Qualität von Tierhäuten dargestellt. Die Tierhäute 11 treffen im Wareneingang ein (siehe Feld 22). Sie weisen unterschiedliche Formen und Größen auf. Die Information über die Größe der gelieferten Tierhaut 11 wird in der Regel vom Lieferanten ermittelt und an den Kunden übermittelt. Diese Größe wird als Brutto-Hautfläche A_{B} bezeichnet, denn die Schlechtflächen, die nicht zur Fertigung verwendet werden dürfen, wurden noch nicht von der Gesamtfläche abgezogen. Die einzelnen Tierhäute 11 enthalten eine Nummer des Lieferanten. Es kann nun bei der Erstellung eines tierhautspezifischen Datensatzes eine neue Nummer vergeben werden, der die Nummer des Lieferanten zugeordnet wird, oder die Nummer des Lieferanten wird beibehalten.

Anschließend erfolgt das erfindungsgemäße Verfahren, welches in Fig. 1 durch einen gestrichelten Kasten gekennzeichnet und mit dem Bezugszeichen 23 bezeichnet ist. Bei dem Verfahren wird zunächst jede Tierhaut 11 digitalisiert, so dass als Ergebnis ein digitales Bild der Tierhaut 11 vorliegt, welchem Daten über die Tierhaut 11 entnommen werden können.

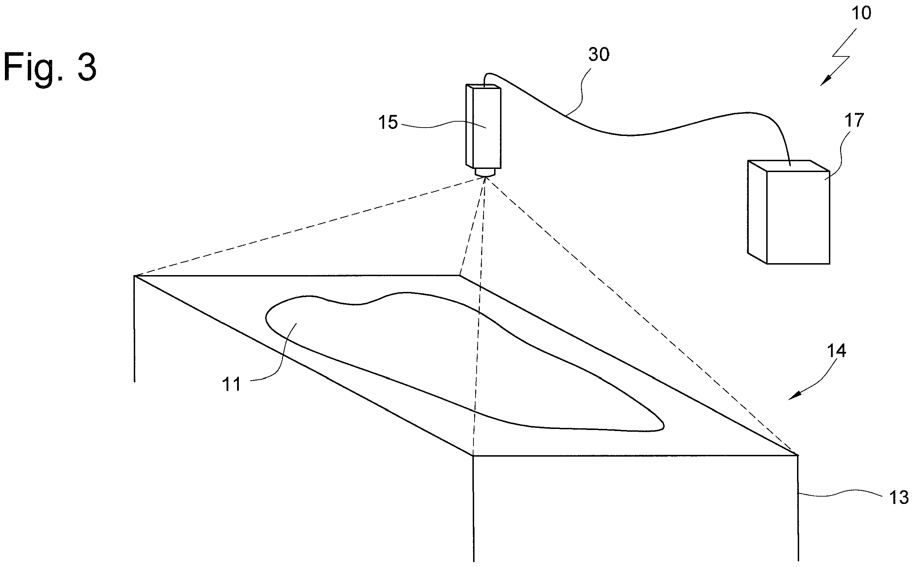

Die Digitalisierung (siehe Bezugszeichen 24 in Fig. 1) erfolgt mittels einer Vorrichtung 10, die in Fig. 3 dargestellt ist. Mit der Vorrichtung 10 wird ein Bild von jeder Tierhaut 11 erzeugt. Die jeweilige Tierhaut 11 wird auf einen Tisch 13 einer Auflagevorrichtung 14 aufgelegt und ggf. aufgespannt. Mit einer Kamera 15 wird ein digitales Bild 16 von der Tierhaut 11 erstellt und mit einem Datenkabel 30 von der Kamera auf einen Computer 17 übertragen. In dem Computer 17 wird das Bild 16 mit einer Software verarbeitet und gespeichert.

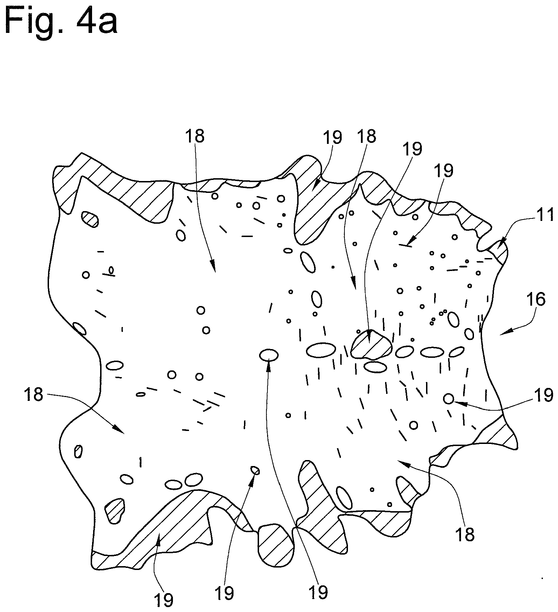

Dann erfolgt eine Fehlererfassung (siehe Bezugszeichen 25 in Fig. 1) und die Unterteilung in Gutflächen 18 und Schlechtflächen 19. Im vorliegenden Ausführungsbeispiel kann die Software die Schlechtflächen 19 der Tierhaut 11 anhand des Bildes 16 automatisch erkennen und daraus die Gutflächen 18 und die Schlechtflächen 19 ermitteln. D.h., dass der Computer 17 ermittelt, welche Flächenausdehnung und Form die Schlechtflächen und die Gutflächen bezogen auf die Form und Fläche der Tierhaut 11 haben. Die Schlechtflächen 19 werden z.B. von der Software auf dem Bild 16 gekennzeichnet, so dass sie für einen Betrachter sichtbar sind. In Fig. 4a ist ein Bild 16 der Tierhaut 11 dargestellt, auf welchem die Schlechtflächen 19 und die Gutflächen 18 erkennbar sind.

Es erfolgt dann das sogenannte Standard-Nesting (siehe Bezugszeichen 26 in Fig. 1), d.h. der Computer 17 errechnet, wie eine Standard-Flächen-Geometrie 20 ohne Überschneidung in die Gutfläche 18 derart möglichst oft eingepasst werden kann, dass die Tierhaut 11 maximal ausgenutzt wird. Für das Standard-Nesting wird erfindungsgemäß die Standard-Flächengeometrie 20 verwendet, welches keine Information über die für die Produktion tatsächlich benötigten Geometrien der Zuschnitte preisgibt, die als Real-Geometrien bezeichnet werden. Das erfindungsgemäße Nesting wird daher in der Anmeldung als Standard-Nesting bezeichnet, im Gegensatz zu dem Real-Nesting, bei welchem die Real-Geometrien genestet werden. Das Real-Nesting findet nach dem Standard-Nesting statt.

Die Standard-Geometrie 20 kann eine beliebige Geometrie sein, wobei es vorteilhaft ist, wenn die Standard-Geometrie eine Flächengröße aufweist, die beim Standard-Nesting vergleichbare Werte liefert, wie die Geometrien, die später bei dem Nesting der tatsächlichen Zuschnitte verwendet werden. D.h., dass die Standard-Geometrie eine repräsentative Bauteilgeometrie darstellt.

Im vorliegenden Ausführungsbeispiel stellt die Standard-Geometrie eine abstrahierte, bezüglich ihrer Flächengröße an die größten Teile eines Cut-Kits (d.h. einer definierten Anzahl von Tierhäuten 11) angenäherte Form dar. Denn wenn die verarbeiteten Flächenzuschnitte eher klein sind, kann die Gutfläche 18 in der Regel besser ausgenutzt werden, als wenn die Flächenzuschnitte groß sind. Im vorliegenden Ausführungsbeispiel entspricht die Flächengröße der größten Teile eines bestimmten Flächenzuschnitts etwa der Flächengröße eines DIN A4 Flächen-Formats. Die Standard-Geometrie 20 ist daher von einem rechteckigen DIN A4-Format gebildet.

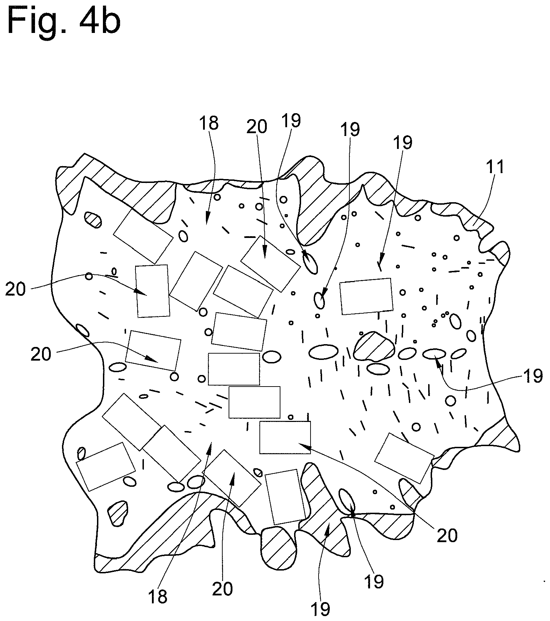

Das Ergebnis des Standard-Nestings wird gespeichert, d.h. ein Bild 16 enthaltend die Schlechtflächen 19 und die eingelagerten Standard-Geometrien 20 wird gespeichert und dem Code zugeordnet, der mit der jeweiligen Tierhaut 11 verbunden ist. Ein Bild 16, auf welchem sowohl die Gutflächen 18, als auch die Schlechtflächen 19 erkennbar sind und zudem das Ergebnis des Standard-Nestings in Form eingelagerter Standard-Geometrien 20 dargestellt ist, ist in Fig. 4b dargestellt. Dieses Bild 16 wird für jede Tierhaut 16 gespeichert.

Mit dem Bezugszeichen 27 in Fig. 1 ist der Verfahrensschritt der Verhältnisbildung bezeichnet. Dabei wird das Verhältnis der Anzahl genesteter Standard-Geometrien 20 zu der Bruttofläche der Tierhaut 11 gebildet.

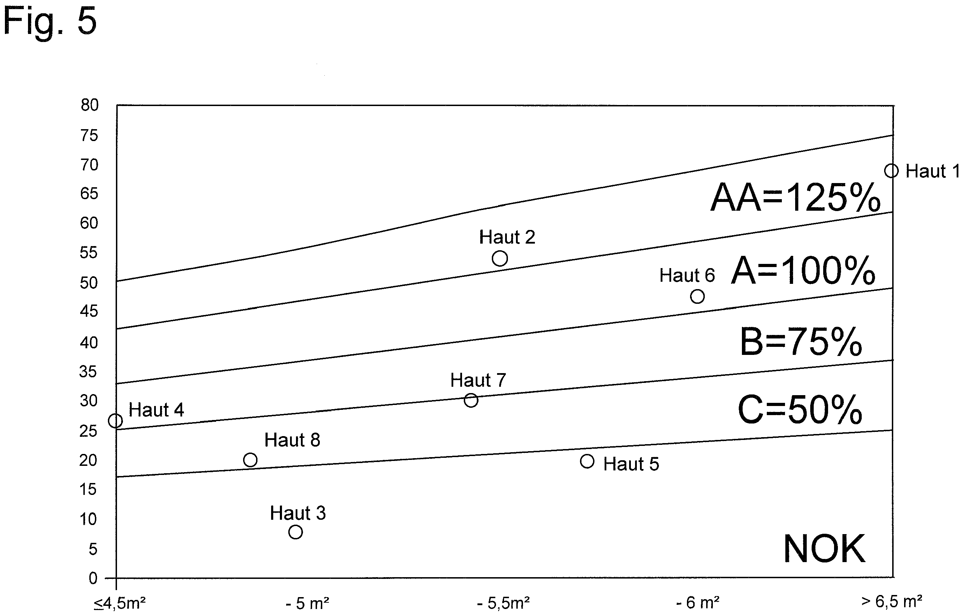

Dann wird der ermittelte Wert des Verhältnisses mit einem in Fig. 5 dargestellten Bewertungsschema verglichen, aus welchem die Qualitätsstufe entnehmbar ist (siehe auch Bezugszeichen 28 in Fig. 1).

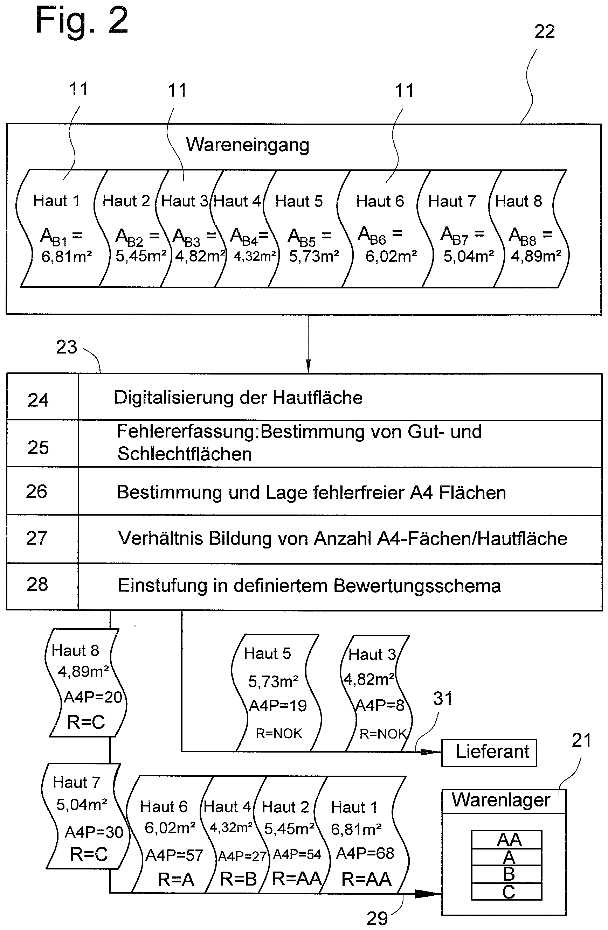

Mit Hilfe von Fig. 2 wird nachfolgend der Verfahrensablauf detaillierter anhand von Beispielen unterschiedlicher Tierhäute 11 beschrieben werden. Im Wareneingang 22 gehen die Tierhäute 11 Nr. 1 bis Nr. 8 ein. Tierhaut 1 (in den Fig. kurz als Haut 1 bezeichnet) hat eine Bruttofläche von 6,81 m2, Tierhaut Nr. 2 (in den Fig. kurz als Haut 2 bezeichnet) eine Bruttofläche von 5,45 m2, Tierhaut 3 eine Bruttofläche von 4,82 m2 usw. Die Bruttoflächen sind in Fig. 2, Bezugszeichen 22 unter der jeweiligen Nummerierung der Tierhaut 11 mit AB1 bis AB8 bezeichnet.

In dem vorliegenden Ausführungsbeispiel hat Tierhaut 11 Nr. 1 eine Größe von 6,81 m2. Beim Standard-Nesting konnten 68 Standard-Geometrien auf der Gutfläche 18 angeordnet werden. In dem Bewertungsfeld gemäß Fig. 5 sind an dem horizontalen Strahl die Größe der Tierhaut und an dem vertikalen Strahl die Anzahl genesteter Standard-Geometrien angegeben. Die Tierhaut 11 Nr. 1 fällt in dem Bewertungsfeld gemäß Fig. 5 in den Bereich der Brutto-Hautfläche ≥6,5 m2. Aus dem Bewertungsschema ist bei 68 genesteten Standard-Geometrien 20 abzulesen, dass die Tierhaut 11 in den Qualitätsbereich AA einzustufen ist.

Tierhaut 11 Nr. 7 (in den Fig. als "Haut 7" bezeichnet) hat eine Brutto-Hautfläche von 5,04 m2. Auf der Gutfläche 18 konnten 30 Standard-Geometrien 20 angeordnet werden. Die Tierhaut 11 Nr. 7 word damit in den Bereich C eingestuft.

Auf der Tierhaut 11 Nr. 5 (in den Fig. als "Haut 5" bezeichnet) mit einer Brutto-Hautfläche von 5,73 m2 konnten 19 Standard-Geometrien 20 angeordnet werden. Damit wird die Tierhaut 11 Nr. 5 in den Ausschuss-Bereich eingestuft, welcher in dem Bewertungs-Feld gemäß Fig. 5 mit NOK bezeichnet ist. Die Tierhäute 11, die in den NOK-Bereich fallen, in diesem Ausführungsbeispiel die Tierhäute 11 Nr. 3 und Nr. 5 (siehe Bezugszeichen 31 in Fig. 2), werden an den Lieferanten zurückgegeben.

Die Tierhäute 11 mit Qualitätsstufen von AA, A, B oder C werden, nachdem sie z.B. mit einem Code markiert wurden (siehe Verfahrensschritt 12), welcher einem hinterlegten Datensatz für die entesprechende Tierhaut 11 zugeordnet ist, in ein Warenlager 21 verbracht (siehe Verfahrensschritt 29) und dort z.B. nach Qualität der Tierhäute 11 oder nach Codenummern separat gelagert.

Für jede von dem Lieferanten gelieferte Tierhaut 11, kann mit dem erfindungsgemäßen Verfahren auch ohne die physische Präsenz der Tierhaut (z.B. weil diese inzwischen verarbeitet wurde) eine Auskunft gegenüber dem Lieferanten gegeben werden, welche Qualität die Tierhaut 11 hatte.

Die Wertebereiche des Bewertungsfeldes nach Fig. 5 werden durch Vereinbarung mit dem Lieferanten ermittelt. Hier wurde z.B. vereinbart, dass eine Tierhaut 11 mit einer Brutto-Hautfläche ≤ 4,5 m2 im NOK-Bereich liegt, wenn 17 oder weniger Standard-Geometrien 20 auf der Gutfläche 18 der Tierhaut 11 angeordnet werden können. Die Tierhaut 11 liegt bei demselben Größenbereich (0-4,5 m2) im Bereich der C-Qualität, wenn zwischen 18 und 25 Standard-Geometrien 20 angeordnet werden können. Für den B-Bereich sind 26 bis 37 Standard-Geometrien notwendig. Von A-Qualität ist die Tierhaut 11, wenn zwischen 38 und 42 Standard-Geometrien 20 auf der Gutfläche 18 implementierbar wären und AA-Qualität erhält die Tierhaut 11, wenn zwischen 43 und 50 Standard-Geometrien 20 auf der Gutfläche 18 Platz finden.

Diese Festlegung wird für jede Grenzgröße, d.h.Brutto-Hautflächen ≤5 m2, ≤5,5 m2, ≤6 m2 und ≤6,5 m2 durchgeführt und die Grenzpunkte werden verbunden. Auf diese Weise ergibt sich für jede Brutto-Hautfläche und für jede Anzahl von Standard-Geometrien 20 ein Qualitätsbereich AA, A, B, C oder NOK. Je nach Vereinbarung könnten natürlich auch feinere oder gröbere Unterteilungen vorgegeben werden, wenn das geeignet ist.

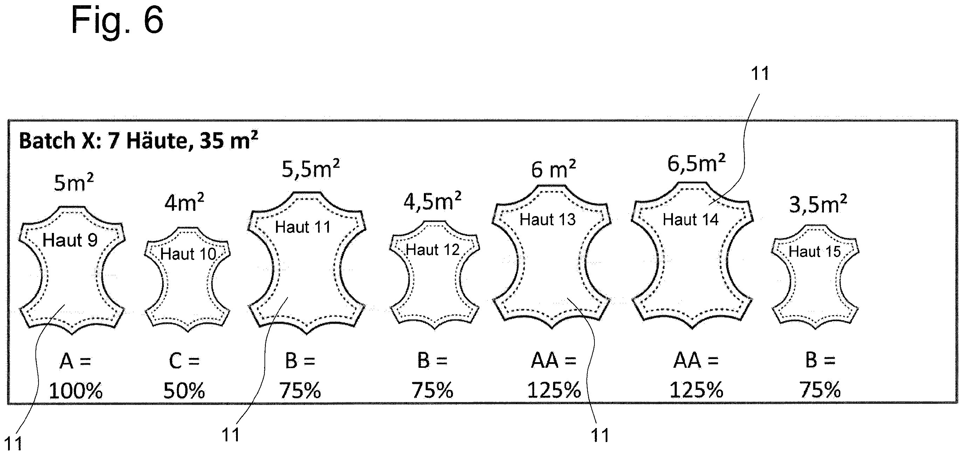

Wenn die Qualitätsstufe jeder Haut ermittelt wurde, kann eine Einpreisung stattfinden, d.h. jeder Qualitätsstufe wird ein Preis zugeordnet. Dazu werden die Tierhäute 11 z.B. zu sogenannten Batches, d.h. Einheiten von mehreren Tierhäuten 11, zusammengefasst. Ein Batch enthält z.B. 7 Häute (siehe Fig. 6). Für eine Qualitätsstufe von 100 % wurde ein bestimmter Preis vereinbart. Die Qualitätsstufe AA erhält z.B. einen bezüglich der Qualitätsstufe 100 % um 25 % höheren Preis. Die Qualitätsstufe B erhält einen um 25 % geminderten Preis und die Qualitätsstufe C erhält einen um 50 % geminderten Preis.

In Fig. 6 ist die Qualitätsstufe unter der jeweiligen Tierhaut 11 aufgeführt. Die Tierhäute 11 mit den Nummern 9 bis 15 bilden ein Batch, wobei die Tierhäute mit den Nrn. 13 und 14 von AA-Qualität, die Tierhaut Nr. 9 A-Qualität, die Tierhäute Nr. 11, 12 und 15 B-Qualität und die Tierhaut Nr. 10 C-Qualität aufweist. Die Flächengrößen der Tierhäute 11 der jeweiligen Qualitätsstufe eines Batches werden zusammengezählt. Das Batch weist 12,5 qm AA-Qualität, 5 qm A-Qualität, 13,5 qm B-Qualität und 4 qm C-Qualität auf. Die Software errechnet nun z.B. aus diesen Werten den an den Lieferanten zu zahlenden Preis. Wenn beispielsweise für 1 qm A-Qualität ein Preis von 40 Euro pro qm vereinbart wurde, ergibt sich für das Batch gemäß Fig. 6 folgende Rechnung:

| | |
|---|---|
| AA | 12,5 x 40 x 1,25 = 625 Euro |
| A | 5 x 40 x 1,0 = 200 Euro |
| B | 13,5 x 40 x 0,75 = 405 Euro |
| C | 4 x 40 x 0,5 = 80 Euro |

Insgesamt ergibt sich also für das Batch ein Gesamtpreis von 1.310,00 Euro.

Wenn zeitlich nach dem Standard-Nesting das Real-Nesting stattfindet, kann die Tierhaut mit den geeigneten Daten aus dem Lager 21 entnommen werden.

## Patentansprüche

1. Verfahren zur Qualitätseinstufung von Tierhäuten umfassend folgende Verfahrensschritte:
- Erfassung der Fläche (A_{B}) der Tierhaut (11) mittels einer Kamera und Analyse der Schlechtflächen (19) und Gutflächen (18),
- Übernahme der Daten in einen Datenspeicher,
**gekennzeichnet dadurch, dass**
- ein Nesting im Sinne einer Anordnung möglichst vieler Geometrien auf der Gutfläche (18) der Tierhaut (11) durchgeführt wird,
- wobei das Nesting als Standard-Nesting durchgeführt wird, bei welchem jede genestete Geometrie von einer Standard-Geometrie (20) mit gleicher Flächengröße und gleicher Form gebildet ist, wobei die Standard-Geometrie eine abstrahierte, von der Realgeometrie abweichende Form darstellt,
- wobei die Tierhaut (11) unter Verwendung des Standard-Nesting-Ergebnisses in eine Qualitätsstufe eingeordnet wird und
- dass später ein Real-Nesting stattfindet, wobei die Realgeometrie eine von der Standard-Geometrie abweichende Geometrie ist, welche in einem Real-Nesting als in der Produktion benötiger Teilezuschnitt Verwendung findet und wobei bei dem Real-Nesting eine oder mehrere gleiche oder unterschiedliche Real-Geometrien in Form einer Fläche auf der fehlerfreien Gutfläche der Tierhaut ohne Überschneidung angeordnet werden.

2. Verfahren zur Qualitätseinstufung von Tierhäuten (11) nach Anspruch 1, **dadurch gekennzeichnet, dass** die Standard-Geometrie von einem Teilkreis, einem Kreis, einem Dreieck, einem Viereck, insbesondere einem Rechteck, oder einem Polygon gebildet ist.

3. Verfahren zur Qualitätseinstufung von Tierhäuten (11) nach Anspruch 1 oder 2, **dadurch gekennzeichnet, dass** ein Quotient aus der Anzahl der im Standard-Nesting auf der Gutfläche (18) der Tierhaut (11) angeordneten Standard-Geometrien (20) und der Größe der Hautfläche (A_{B}) gebildet wird.

4. Verfahren zur Qualitätseinstufung von Tierhäuten (11) nach Anspruch 3, **dadurch gekennzeichnet, dass** eine Einordnung der Tierhaut (11) in eine Qualitätsstufe mit Hilfe des Quotienten erfolgt.

5. Verfahren zur Qualitätseinstufung von Tierhäuten (11) nach Anspruch 4, **dadurch gekennzeichnet, dass** die ermittelte Qualitätsstufe einem Datensatz der Tierhaut (11) zugeordnet wird, welcher ein Bild (16) des Standard-Nestings und / oder tierhautbezogene Daten enthält.

6. Verfahren zur Qualitätseinstufung von Tierhäuten (11) nach Anspruch 5, **dadurch gekennzeichnet, dass** der Datensatz unter einem der jeweiligen Tierhaut (11) zugeordneten Code gespeichert wird.

7. Verfahren zur Qualitätseinstufung von Tierhäuten (11) nach einem der Ansprüche 4 bis 6, **dadurch gekennzeichnet, dass** Tierhäute (11), welche die Mindest-Qualitätsstufe nicht erfüllen, ausgesondert werden.

8. Verfahren zur Qualitätseinstufung von Tierhäuten (11), **dadurch gekennzeichnet, dass** Tierhäute (11), deren Qualität mindestens der Mindest-Qualitätsstufe entspricht, in ein Lager überführt werden.

9. Verfahren zur Qualitätseinstufung von Tierhäuten (11) nach einem der vorangehenden Ansprüche, **dadurch gekennzeichnet, dass** die Erfassung der Schlechtfläche (19) und der Gutfläche (18) und / oder das Nesting mittels einer Vorrichtung (10) gemäß einem der Ansprüche 10 oder 11 vorgenommen wird.

10. Vorrichtung zur Durchführung des Verfahrens nach einem der Ansprüche 1 bis 10, umfassend einen Tisch (13) zum Auflegen einer Tierhaut (11), eine Kamera (15) zur Erstellung eines Bildes (16) der Tierhaut (11) und einen Computer (17) mit wenigstens einer darauf betriebenen Software, welcher mit der Kamera (15) über ein Datenkabel (30) verbunden ist und mit welchem die Bilddaten mittels wenigstens einer auf dem Computer betriebenen Software verarbeitbar sind derart, dass Gutflächen (18) und Schlechtflächen (19) der Tierhaut (11) erfassbar und ein Nesting durchführbar ist, **dadurch gekennzeichnet, dass** mittels des Computers (17) und der Software ein Standard-Nesting durchführbar ist, bei welchem jede genestete Geometrie von einer Standard-Geometrie (20) mit gleicher Flächengröße und gleicher Form gebildet ist, wobei die Standard-Geometrie eine abstrahierte, von der Realgeometrie abweichende Form darstellt, dass bei dem Standard-Nesting die Standard-Geometrie möglichst oft auf der Gutfläche (18) der Tierhaut (11) angeordnet wird und dass die Tierhaut (11) unter Verwendung des Standard-Nesting-Ergebnisses in eine Qualitätsstufe einordenbar ist.

11. Vorrichtung zur Qualitätseinstufung von Tierhäuten (11) nach Anspruch 10, **dadurch gekennzeichnet, dass** mittels der Vorrichtung die Schlechtflächen (19) unmittelbar aus dem erstellten Bild (16) erkennbar oder alternativ eine manuelle Kennzeichnung der Schlechtflächen (19) aus dem erstellten Bild (16) erkennbar und nach Form, Größe und Position relativ zu der Form, Größe und Position der Fläche (A_{B}) der Tierhaut (11) anordenbar und speicherbar sind.

## Claims

1. Method for quality classification of animal skins, comprising the following method steps:
- recording the surface area (A_{B}) of the animal skin (11) by means of a camera and analysing the faulty areas (19) and good areas (18),
- transferring the data to a data storage device,
**characterised in that**
- nesting, in the context of an arrangement, is carried out on as many geometries as possible on the good area (18) of the animal skin (11),
- wherein the nesting is carried out as standard nesting, with which each nested geometry is formed by a standard geometry (20) having the same surface area and shape, wherein the standard geometry represents an abstract shape differing from the real geometry,
- wherein the animal skin (11) is classified into a quality level using the standard nesting result, and
- real nesting takes place later, wherein the real geometry is geometry differing from the standard geometry, which is used in real nesting as part cutting required in production, and wherein, in the real nesting, one or more identical or different real geometries are arranged in the shape of a surface area on the fault-free good area of the animal skin, without overlapping.

2. Method for quality classification of animal skins (11) according to claim 1, **characterised in that** the standard geometry is formed of a pitch circle, a circle, a triangle, a quadrilateral, in particular a rectangle, or a polygon.

3. Method for quality classification of animal skins (11) according to claim 1 or 2, **characterised in that** a quotient is formed from the number of standard geometries (20) arranged in the standard nesting on the good area (18) of the animal skin (11) and the size of the skin surface area (A_{B}).

4. Method for quality classification of animal skins (11) according to claim 3, **characterised in that** a classification of the animal skin (11) into a quality level takes place with the aid of the quotient.

5. Method for quality classification of animal skins (11) according to claim 4, **characterised in that** the determined quality level is assigned to a data set of the animal skin (11), which contains an image (16) of the standard nesting and/or animal skin-related data.

6. Method for quality classification of animal skins (11) according to claim 5, **characterised in that** the data set is stored under a code assigned to the respective animal skin (11).

7. Method for quality classification of animal skins (11) according to one of claims 4 to 6, **characterised in that** the animal skins (11) that do not fulfil the minimum quality level are discarded.

8. Method for quality classification of animal skins (11), **characterised in that** animal skins (11) whose quality corresponds to at least the minimum quality level are transferred into stock.

9. Method for classification of animal skins (11) according to one of the preceding claims, **characterised in that** the detection of faulty areas (19) and of good areas (18) and/or the nesting is performed by means of a device (10) according to one of claims 10 or 11.

10. Device for performing the method according to one of claims 1 to 10, comprising a table (13) to place an animal skin (11) on, a camera (15) for recording an image (16) of the animal skin (11), and a computer (17) having at least one software operated thereon, which is connected to the camera (15) via a data cable (30) and with which the image data can be processed by means of at least one piece of software operated on the computer, in such a way that good areas (18) and faulty areas (19) of the animal skin (11) can be recorded and nesting can be performed, **characterised in that** standard nesting can be performed by means of the computer (17) and the software, with which each nested geometry is formed by a standard geometry (20) having the same surface area and same shape, wherein the standard geometry represents an abstract shape differing from the real geometry, with the standard nesting the standard geometry is arranged as often as possible on the good area (18) of the animal skin (11) and the animal skin (11) can be classified into a quality level using the standard nesting results.

11. Device for the quality classification of animal skins (11) according to claim 10, **characterised in that**, by means of the device, the faulty areas (19) can be recognised directly from the generated image (16) or alternatively manual marking of the faulty areas (19) can be recognised from the generated image (16) and can be arranged and stored according to shape, size and position relative to the shape, size and position of the surface area (A_{B}) of the animal skin (11).

## Revendications

1. Procédé de classification qualitative de peaux d'animaux, comprenant les étapes de procédé suivantes :
- saisir la surface (As) d'une peau d'animal (11) par l'intermédiaire d'une caméra et analyser les surfaces de mauvaise qualité (19) et les surfaces de bonne qualité (18), et
- transférer ces données à l'intérieur d'une mémoire de données,
**caractérisé en ce que**
- une imbrication est réalisée au sens d'un agencement du plus grand nombre possible de géométries sur la surface de bonne qualité (18) de la peau d'animal (11),
- l'imbrication est réalisée sous la forme d'une imbrication standard, dans laquelle chaque géométrie imbriquée est formée par une géométrie standard (20) de même taille de surface et de même forme, la géométrie standard représentant une forme abstraite, différente de la géométrie réelle,
- la peau d'animal (11) est classée à un niveau qualitatif en utilisant le résultat de l'imbrication standard, et **en ce que**
- une imbrication réelle est ensuite réalisée, dans laquelle la géométrie réelle est une géométrie s'écartant de la géométrie standard, qui est utilisée dans une imbrication réelle en tant que découpe de pièce nécessaire à la production, et une ou plusieurs géométrie(s) réelle(s) identique(s) ou différente(s) est/sont disposée(s) lors de l'imbrication réelle sous la forme d'une surface sur la surface de bonne qualité exempte de défauts de la peau d'animal, sans chevauchement.

2. Procédé de classification qualitative de peaux d'animaux (11) selon la revendication 1, **caractérisé en ce que** la géométrie standard est formée par un arc de cercle, un cercle, un triangle, un quadrilatère, en particulier un rectangle, ou un polygone.

3. Procédé de classification qualitative de peaux d'animaux (11) selon la revendication 1 ou 2, **caractérisé en ce qu'**un quotient est établi à partir du nombre de géométries standard (20) disposées dans l'imbrication standard sur la surface de bonne qualité (18) de la peau d'animal (11) et de la taille de la surface de peau (A_{B}).

4. Procédé de classification qualitative de peaux d'animaux (11) selon la revendication 3, **caractérisé en ce qu'**un classement de la peau d'animal (11) à un niveau qualitatif est réalisé à l'aide du quotient.

5. Procédé de classification qualitative de peaux d'animaux (11) selon la revendication 4, **caractérisé en ce que** le niveau qualitatif déterminé est associé à un ensemble de données de la peau d'animal (11) contenant une image (16) de l'imbrication standard et / ou des données relatives à la peau d'animal.

6. Procédé de classification qualitative de peaux d'animaux (11) selon la revendication 5, **caractérisé en ce que** l'ensemble de données est enregistré sous un code associé à la peau d'animal (11) concernée.

7. Procédé de classification qualitative de peaux d'animaux (11) selon l'une quelconque des revendications 4 à 6, **caractérisé en ce que** les peaux d'animaux (11) qui ne satisfont pas au niveau qualitatif minimal sont écartées.

8. Procédé de classification qualitative de peaux d'animaux (11), **caractérisé en ce que** les peaux d'animaux (11) dont la qualité est au moins conforme au niveau qualitatif minimal sont transférées dans un lieu de stockage.

9. Procédé de classification qualitative de peaux d'animaux (11) selon l'une quelconque des revendications précédentes, **caractérisé en ce qu'**il est procédé à la saisie de la surface de mauvaise qualité (19) et de la surface de bonne qualité (18) et/ou à l'imbrication, au moyen d'un dispositif (10) selon l'une quelconque des revendications 10 ou 11.

10. Dispositif pour la mise en oeuvre du procédé selon l'une quelconque des revendications 1 à 10, comprenant une table (13) pour poser une peau d'animal (11), une caméra (15) pour la réalisation d'une image (16) de la peau d'animal (11) et un ordinateur (17) contenant au moins un logiciel fonctionnant sur celui-ci, qui est relié à la caméra (15) par l'intermédiaire d'un câble de données (30) et avec lequel des données d'image peuvent être traitées à l'aide d'au moins un logiciel exécuté sur l'ordinateur de telle sorte que des surfaces de bonne qualité (18) et des surfaces de mauvaise qualité (19) d'une peau d'animal (11) puissent être saisies et qu'une imbrication puisse être réalisée, **caractérisé en ce qu'**une imbrication standard peut être réalisée au moyen de l'ordinateur (17) et du logiciel, dans lequel chaque géométrie imbriquée est formée par une géométrie standard (20) qui présente la même taille de surface et la même forme, la géométrie standard représentant une forme abstraite, différente de celle de la géométrie réelle, **en ce que**, lors de l'imbrication standard, la géométrie standard est disposée le plus souvent possible sur la surface de bonne qualité (18) de la peau d'animal (11), et **en ce que** la peau d'animal (11) peut être classée à un niveau qualitatif en utilisant le résultat de l'imbrication standard.

11. Dispositif de classification qualitative de peaux d'animaux (11) selon la revendication 10, **caractérisé en ce que**, au moyen du dispositif, les surfaces de mauvaise qualité (19) sont directement reconnaissables à partir de l'image réalisée (16) ou, alternativement, une caractérisation manuelle des surfaces de mauvaise qualité (19) est reconnaissable à partir de l'image réalisée (16) et est agençable et mémorisable suivant la forme, la taille et la position par rapport à la forme, la taille et la position de la surface (As) de la peau d'animal (11).
